(19) Europäisches Patentamt
European Patent Office
Office européen des brevets

(11) Publication number: 0 045 118 B1

(12) # EUROPEAN PATENT SPECIFICATION

(45) Date of publication of patent specification: 04.04.84

(51) Int. Cl.³: C 07 C 59/11, C 07 C 59/205, C 07 C 59/46, C 07 C 59/80 //C07C177/00

(21) Application number: 81201031.2

(22) Date of filing: 04.01.80

(60) Publication number of the earlier application in accordance with Art. 76 EPC: 0013607

(54) **Intermediates for the synthesis of bicyclo (2,2,1) heptanes and bicyclo (2,2,1) hept-2Z-enes.**

(30) Priority: 05.01.79 GB 7900368

(43) Date of publication of application: 03.02.82 Bulletin 82/5

(45) Publication of the grant of the patent: 04.04.84 Bulletin 84/14

(84) Designated Contracting States: CH DE FR GB IT NL SE

(56) References cited:
FR - A - 2 316 926

PROSTAGLANDINS, vol. 11, no. 6, June 1976, pages 953-960 T.J. LEENEY et al.: "Inhibitors of *prostaglandin biosynthesis. A bicyclo-*(2.2.1)-heptene analogue of '2' series prostaglandins and related derivatives"

(73) Proprietor: NATIONAL RESEARCH DEVELOPMENT CORPORATION
101 Newington Causeway
London SE1 6BU (GB)

(72) Inventor: Jones, Robert Leslie
62 Caiystane Gardens
Edinburgh EH10 6SY (GB)
Inventor: Wilson, Norman Happer
47 West Saville Terrace
Edinburgh EH9 3DP (GB)

(74) Representative: Stephenson, Gerald Frederick
Patent Department National Research Development Corporation 101 Newington Causeway
London SE1 6BU (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 045 118 B1

# Intermediates for the synthesis of bicyclo[2,2,1]heptanes and bicyclo[2,2,1]hept-2Z-enes

This invention relates to intermediates for use in the synthesis of bicyclo[2,2,1]heptanes and hept-2Z-enes.

The document "Prostaglandins" vol. 11 (1976) pp. 953—60 describes a group of 5,6-disubstituted bicyclo[2,2,1]heptanes and hept-2Z-enes, some of which are reported to be inhibitors of prostaglandin synthesis. In particular bicyclo[2,2,1]hept-2Z-enes substituted at the 5-endo position by a 6-carboxyhex-2Z-enyl group and at the 6-exo position by a formyl or dimethoxymethyl group are disclosed as intermediates. The last mentioned compound is reported to be a relatively weak inhibitor of prostaglandin synthesis.

FR—A—2 316 926 discloses bicyclo[2,2,1]hept-2Z-enes substituted at the 5-exo position by a 6-carboxymethyl hex-2Z-enyl group and at the 6-endo position by a hydroxymethyl or formyl group, as intermediates.

In European Applications 80 300039.7 and 80 300040.5 (published as EP—A1—0 013 607 and EP—A1—0 013 608, respectively) a group of 5,6-disubstituted bicyclo[2,2,1]heptanes and hept-2Z-enes is described and claimed, these compounds being of value for use in pharmaceutical compositions particularly in the context of the inhibition of thromboxane activity. The compounds of the present invention constitute intermediates in a synthetic route of particular value for the preparation of a particular sub-group of such biologically active bicyclo[2,2,1]heptanes and hept-2Z-enes. This sub-group comprises those compounds having a group —C(R)=N— at the 6-position in which R is an aliphatic hydrocarbon residue or an aliphatic hydrocarbon residue substituted directly or through an oxygen or sulphur atom by an aromatic residue.

Accordingly the present invention comprises a compound being a bicyclo[2,2,1]heptane or bicyclo[2,2,1]hept-2Z-ene which is substituted at the 5-position by a 6-carboxyhex-2-enyl group or a modification thereof in which the group is altered by one, or where appropriate by an appropriate combination, of two or more of the following:
(a) reduction of the double bond optionally accompanied by replacement of a carbon atom at the 1, 2 or 3 position by a sulphur or oxygen atom (b) alteration of the position of the double bond; (c) shortening or lengthening of the carbon chain by one or two methylene groups; and (d) formation of an amide, ester or salt derivative of the carboxy group; and at the 6-position by a group

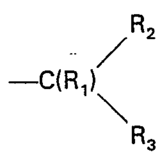

in which $R_1$ represents an aliphatic hydrocarbon residue or an aliphatic hydrocarbon residue substituted directly or through an oxygen or sulphur atom by an aromatic residue, $R_2$ represents hydrogen and $R_3$ represents a hydroxy group, or $R_2$ and $R_3$ together represent the oxygen atom of a carbonyl group.

The system used herein for numbering the bicyclo[2,2,1]heptane and hept-2Z-ene ring systems is illustrated hereinafter.

It will be seen that the modifications of the 6-carboxyhex-2-enyl group which may be made in compounds according to the present invention are of two types. Thus, the modification either involve the hex-2-enyl group or the 6-carboxy group. Among modifications of the first form, which are listed under (a) to (c) above, certain preferences may be indicated. Thus, where the double bond is reduced and a carbon atom replaced, the replacement is conveniently by an oxygen rather than a sulphur atom and also is conveniently at the 2 or 3 position (the 2 and 3 positions referred to the carbon atom of the 6-carboxyhex-2-enyl group attached to the ring system as position 1 correspond, respectively, to positions 5 and 6 when referred to the carboxylic acid group as $C_1$). Moreover, where the position of the double bond is altered, this is conveniently to the 3,4 position (i.e. the 4, 5 position if referred to the $C_1$ of the carboxylic acid group) and where the carbon chain is shortened or lengthened this is conveniently at the end of the chain adjacent to the carboxy group. Among the second form of modification, listed under (d) above, examples of amide, ester and salt derivatives of particular interest are to be found in the prostaglandin art and include esters such as alkyl esters, amides such as those containing the group $—CONHSO_2CH_3$ and variants thereon, and salts with various physiologically acceptable cations. Specific examples of salts are those formed with an alkali metal such as sodium or with quaternary ammonium ions or amines such as tris (the symbol tris represents the compound 2-amino-2-hydroxymethylpropane-1,3-diol).

Examples of specific substituent groups at the 5-position of the bicyclo[2,2,1]heptane or hept-2Z-ene include, in addition to $—CH_2—CH=CH—(CH_2)_3CO_2H$, the group $—(CH_2)_6CO_2H$, as well as amide, salt and particularly ester derivatives of both groups.

The aliphatic and araliphatic groups $R_1$ present in the 6-substituent of compounds according to the present invention generally correspond to those groups $R_1$ present in the 6-substituent of the

bicyclo[2,2,1]heptanes and hept-2Z-enes of Application 80 300039.7 and which are described in detail in that application. Thus, for example, $R_1$ may conveniently be an aliphatic hydrocarbon residue of one to five, six, seven, eight, nine, ten or even more carbon atoms in substituted, especially directly substituted, form or particularly in unsubstituted form. Examples of $R_1$ thus include branched or unbranched alkyl groups such as methyl, ethyl, propyl, butyl, amyl and araliphatic groups derived therefrom. Groups $R_1$ being or comprising an aliphatic hydrocarbon residue of 1 to 3 carbon atoms are of some special interest, for example ethyl and particularly methyl. Aromatic residues present in groups $R_1$ are described in detail in Application 80 300039.7 and may be hydrocarbon or heterocyclic residues, for example phenyl and substituted phenyl groups and pyridyl groups such as pyrid-1-yl.

It will be appreciated that the structures of the compounds described above provide various opportunities for the occurrence of isomerism although the double bond of the unsaturated ring system is of the Z configuration. The substituents at the 5 and 6 positions of the ring may be in the *cis* or *trans* relationship to each other, compounds of the latter configuration being preferred. Moreover, as the compounds contain a bridged ring system, then different isomers will exist which vary according to the way in which the 5- and 6-substituents are disposed in relation to the bridging methylene group. Isomers of particular interest are (illustrated for the unsaturated ring system):

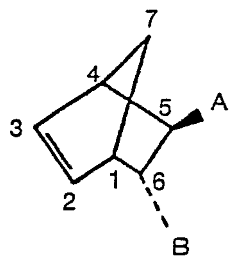

*5-exo, 6-endo*

and, more particularly,

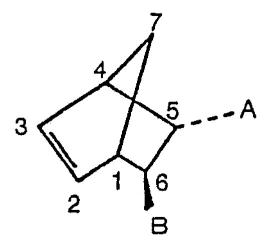

*5-endo, 6-exo*

The system of nomenclature used herein for the bicyclo[2,2,1]heptane and hept-2Z-ene ring systems is indicated in the above formulae. It should be noted that the bridged ring systems are numbered in such a way that, starting with one of the bridging positions as position 1, the double bond in the unsaturated ring system receives the lowest number possible (2), the 6-carboxyhex-2-enyl group or a modification thereof (represented as A) then being at the 5-position and the grouping

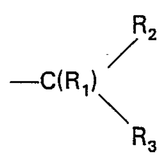

(represented as B) being at the 6-position. For conformity, a similar system of numbering is followed for the saturated ring system.

Where the substituent at the 5-position of the ring is a 6-carboxyhex-2-enyl group or a modification thereof which still contains the double bond then the configuration about this bond is preferably *cis* (Z) rather than *trans* (E). In addition to the types of isomerism already described, optical isomerism is possible. More usually, however, the compounds of this invention are used in their racemic form. It will be appreciated that the formulae used above to illustrate the 5-exo, 6-endo and 5-endo, 6-exo isomers show one of the two enantiomers which exist, the other having a structure which is the mirror image of that illustrated.

Compounds according to the present invention may conveniently be prepared by using as a starting material a compound containing the unsaturated ring system and having substituents on the ring system which are suitable precursors for those in the final compound. The formation of such an unsaturated bicyclic ring system is conveniently effected by means of a Diels-Alder reaction. Compounds containing the saturated ring system may then conveniently be produced by reduction of the ring double bond, for example by the use of hydrogen in the presence of a catalyst such as palladium-charcoal, such reduction more usually being effected prior to modification of the substituents. One convenient starting material providing suitable precursors for the final substituents which may be utilised in the preparation of many of the compounds according to the present invention is a maleinaldehydic acid pseudo ester of formula

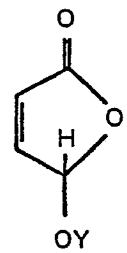

wherein Y represents a hydrocarbon residue, preferably an aliphatic residue such as methyl or especially ethyl. Following reaction of this compound with cyclopentadiene in a Diels-Alder reaction, modification of the substituents at the 5- and 6-positions of the resulting bicyclic ring system is effected to give at the 5-position a 6-carboxyhex-2-enyl group or a modification thereof and at the 6-position a formyl group, which may then be reacted further to provide a group

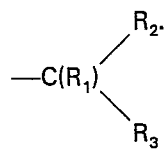

An example of such a procedure for the preparation of 6-formyl substituted compounds is shown in the reaction scheme at the end of the specification following the examples. The numbering of the compounds used in this scheme corresponds to that used hereinafter in the Examples under sections (A) and (B) of the description of the preparation of 6-formyl substituted starting materials. The following abbreviations are employed in the scheme: Ts, toluene sulphonyl; DMSO, dimethyl sulphoxide; Et, ethyl; Bu, butyl. Although the various bicyclic compounds are for convenience shown in the reaction scheme by a structure which represents shown in the reaction scheme by a structure which represents one of the two enantiomeric forms in which they exist, these compounds are generally obtained as a racemic mixture of the two enantiomers. In this procedure the use of ethoxycarbonyl rather than methoxycarbonyl groups and of ethyl rather than methyl acetal groups has been found to be of value. In the final stages of the procedure the acetal group of compound (7) is converted to a formyl group to give either compound (8) in which the 5-substituent terminates in a carboxy group in derivative form or compound (8′) in which the 5-substituent terminates in a free carboxy group.

Further reaction of a formyl group at the 6-position of the bicyclo[2,2,1]heptane or hept-2Z-ene ring system is effected with a Grignard reagent, R-Mg-Halogen, to thereby convert the formyl group to a secondary alcohol group,

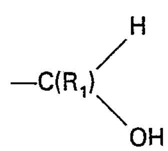

Oxidation of this secondary alcohol, for example with Jones reagent, produces a carbonyl group —$C(R_1)$=O. The compounds according to the present invention containing such a group —$C(R_1)$=O may be reacted with a suitable reagent to produce the biological active compounds of Application 80 300039.7, the reagent conveniently taking the form of a substituted hydroxylamine, $NH_2$—OR′, for example p-fluorobenzyloxyamine hydrochloride. Such a reaction is generally effected in the presence of a base, for example pyridine. When the desired 5-substituent terminates in a free carboxy group, it is possible either to react the reagent $NH_2$—OR′ directly with a compound such as compound (8′) of the scheme shown at the end of the specification following the Examples which contains a free carboxy group or with the corresponding compound in which the carboxy group is protected. Such a protected compound is conveniently obtained from the compound (7), for example by reaction with diazomethane followed by treatment with aqueous acid to give compound (8). Following reaction of the reagent $NH_2$—OR′ with the formyl group of a compound such as (8), the carboxy group is deprotected, for example by de-esterification using $KOH/CH_3OH/H_2O$, the overall yield generally being slightly better than if the carboxy group is not protected.

The compounds of Application 80 300040.5 may be prepared from the compounds of the present invention containing a group —$C(R_1)$=O by reaction with a suitable reagent in an analogous fashion to that described for the compounds of Application 80 300039.7.

Modification of the 6-carboxyhex-2-enyl group may be effected through the initial introduction of the group in modified form or by modification of this group during or at the end of the synthesis of a compound according to the present invention. Thus, ester formation, for example, may conveniently be

effected at the stage indicated hereinbefore and amides may similarly be prepared by conventional procedures. In some cases, however, a modification of type (d) described above, for example salt formation, may be better left until the end of the synthesis of the biologically active compound. Indeed, the procedures for effecting the various modifications indicated above will be apparent from the considerable literature existing on prostaglandin chemistry. Thus, for example, one convenient route for the preparation of compounds containing a 6-carboxyhexyl group involves, in the case of the bicyclo[2,2,1]heptanes, the reduction of the compound (7) shown in the scheme at the end of the specification following the Examples to saturate both the double bond in the ring and that of the 6-carboxyhex-2Z-enyl group. In the case of the bicyclo[2,2,1]hept-2Z-enes the corresponding 5-(6′-carboxyhexyl), 6-formyl compound may be obtained by the Diels-Alder reaction of 8-carboxy-1-formyl-oct-1-ene and cyclopentadiene. (A separation of the two *trans* isomers obtained being required).

It will be appreciated that the methods described above are not the only ones which may be used for the preparation of compounds according to the present invention and that various alternative procedures may be used as will be apparent to those skilled in the art of prostaglandin chemistry.

The invention is illustrated by the following Examples.

## Examples

Although the various compounds have predominantly the isomeric form indicated, some minor contamination by other isomers, for example the 5-endo, 6-endo isomer, may be present. All of the compounds are obtained in the form of a racemic mixture.

Following Example 3 the preparation of the starting material of Example 1, 5-endo-(6′-carboxyhex-2′Z-enyl)-6-exo-formyl-bicyclo[2,2,1]heptane, is described and thereafter the preparations of two alternative starting materials containing a formyl substitutent at the 6-position, 5-endo-(6′-carboxyhex-2′Z-enyl)-6-exo-formyl-bicyclo[2,2,1] hept-2′Z-ene and 5-endo-(6′-carboxyhexyl)-6-exo-formyl-bicyclo[2,2,1]heptane, are also given.

### Example 1

5-endo-(6′-Carboxyhex-2′Z-enyl)-6-exo-(1′-hydroxyethyl)bicyclo[2,2,1]heptane

5-endo-(6′-Carboxyhex-2′Z-enyl)-6-exo-formyl-bicyclo[2,2,1]heptane (250 mg, 1 mmole) is dissolved in dry tetrahydrofuran (10 ml) at 0°C and treated under nitrogen with stirring over 30 minutes with a 1M solution of methyl magnesium iodide in ether (2 ml). The mixture is stirred under nitrogen overnight whilst it is allowed to come to room temperature. The reaction is then quenched by the addition of dilute aqueous hydrochloric acid and the product is extracted with ether (3x); the ether solution is dried and evaporated to give the title compound as an oil (200 mg). A small sample is treated to form the methyl ester trimethylsilyl ether and on gas chromatography mass spectroscopy on a 3% OVI column this shows a carbon value of 18.2, a $M^+$ value of 352 and a base peak at 117. Chromatography of the bulk of the oily product on a 400 mm x 15 mm column of Sephadex LH20 (trade name) substituted with Nedox 1114 (trade name) olefin oxide to 20% w/w (Lipidex — trade name) using a mixture of (all proportions by volume) 100 parts of hexane, 100 parts of 1,2-dichloroethane, 5 parts of ethanol and 0.1% of the total of glacial acetic acid as eluant yields the isomeric secondary alcohols differing in the configuration at the newly introduced asymmetric carbon atom (—$\overset{*}{C}HOH.CH_3$). In $CDCl_3$ these isomeric products give the following $\delta$ values. First isomer eluted: 7.3 (s-broad, 1H, O*H*); 5.45 (m, 2H, olefini H); 3.6 (m-qxd, 1H, C*H*OH); 2.5—1.0 (m, 21H, olefinic H); 1.2 (d, C$H_3$ discernible).

Second isomer eluted: 7.8 (s-broad, 1H, O*H*); 5.4 (m, 2H, olefinic H); 3.55 (m-qxd, 1H, C*H*OH); 2.5—1.0 (m, 18H, aliphatic H); 1.2 (d, C$H_3$ discernible).

### Example 2

5-endo-(6′-Carboxyhex-2′Z-enyl)-6-exo-acetyl-bicyclo[2,2,1]heptane

The procedure described in Example 1 is repeated with 600 mg of the aldehyde to give a mixture of the two isomeric alcohols (500 mg). This mixture is dissolved in pure acetone (15 ml) and the solution is cooled to 0°C. Jones reagent (600 $\mu$l of a solution prepared by dissolving 26.7 g of chromic anhydride in 23 ml of concentrated sulphuric acid and diluting to 100 ml with water, followed by filtration) is added slowly to the cooled solution with vigorous stirring over 15 minutes. After a further 10 minutes stirring at 0°C the mixture is poured into water and the product extracted with ether. The ether solution is dried and evaporated to give the title compound as an oil (about 75% overall yield from the formyl compound). The methyl ester derivative on gas chromatography mass spectroscopy on a 3% OVI column exhibits a carbon value of 17.15, a $M^+$ value of 278 and a base peak of 43/137. NMR spectroscopy in $CDCl_3$ gives the following $\delta$ values: 10.0 (s-broad, 1H, COO*H*); 5.4 (m, 2H, olefinic H); 2.8—1.1 (m, 21H, aliphatic H); 2.2 (s, C$H_3$—CO, discernible).

### Example 3

Use of 5-endo-(6′-Carboxyhex-2′Z-enyl)-6-exo-acetylbicyclo[2,2,1]heptane as an intermediate in the preparation of 5-endo-(6′-Carboxyhex-2′Z-enyl)-6-exo-[1-(p-fluorobenzyl-oxyimino)ethyl]bicyclo-[2,2,1]heptane

5-endo-(6'-Carboxyhex-2'Z-enyl)-6-exo-acetyl-bicyclo [2,2,1]heptane (100 mg), prepared as described in Example 2, is heated with p-fluorobenzyloxyamine hydrochloride (100 mg) in dry pyridine (5 ml) at 60°C for 2 hours. The pyridine is removed in vacuo and the residue is partitioned between water (pH 4) and diethyl ether. The ether is removed in vacuo to give an oil which is purified by liquid-gel partition chromatography on a column of Sephadex LH 20 substituted with Nedox 1114 olefin oxide to 20% w/w, eluting with dichloroethane/hexane/ethanol (100:100:5 v/v/v) containing 0.1% v/v of acetic acid. The chromatography gives the title compound as an almost colourless oil (90 mg), $\lambda_{max}(CH_3OH)$: 263 nm, $\varepsilon_{max}$ 620; M.S.: $M^+$ 401; $\delta(CDCl_3)$: 1.85 (s, about 3H, methyl H), 5.00 (s, 2H, benzyl H), 5.30 (m, 2H, olefinic H), 6.9—7.5 (m, 4H, aromatic H).

PREPARATION OF 6-FORMYL SUBSTITUTED STARTING MATERIALS

(A) Preparation of 5-endo-(6'-Carboxyhex-2'Z-enyl)6-exo-formylbicyclo [2,2,1] heptane

1. *Maleinaldehydic acid pseudo-ethyl ester*

30 g of redistilled furan-2-aldehyde is mixed with 600 ml dry ethanol and 300 mg of methylene blue is added. Dry air is blown gently through the solution and the material is irradiated with a 300 W tungsten lamp for about two days until t.l.c. in a silica gel/ether system shows essentially no remaining starting material. The solution is then stirred with vanadium pentoxide for four hours, filtered, and the solvent removed under reduced pressure. The residual oil is distilled under high vacuum to give the title compound as an oil (23.6 g, 76%), b.p. 90—92°C/0.2 mm.

2. *Diels-Alder reaction between maleinaldehydic acid pseudoethyl ester and cyclopentadiene*

Freshly cracked cyclopentadiene (9.0 g) is mixed with 11.0 g of the pseudo ester (1). A gentle warming is observed and the mixture is allowed to stand overnight. The N.M.R. spectrum typically shows the formation of the adduct (2) to be complete and the material is taken to the next step without purification.

3. *5-endo-Ethoxycarbonyl-6-exo-diethoxymethyl-bicyclo [2,2,1] hept-2Z-ene*

The Diels-Alder adduct (2) (10 g) is heated in a mixture of triethyl orthoformate (10 ml), dry ethanol (100 ml), and concentrated sulphuric acid (1 ml). The mixture darkens and after 12 hours is cooled and treated with anhydrous potassium carbonate (5 g) and ether (150 ml). Water is then slowly added with efficient mixing to neutralise the acid. The product is extracted with ether, washed with water and distilled to give the title compound as an oil (7.3 g, 63%) b.p. 115—120°C/0.3 mm.

3A. *5-endo-Ethoxycarbonyl-6-exo-diethoxymethyl-bicyclo [2,2,1] heptane*

5-endo-Ethoxycarbonyl-6-exo-diethoxymethyl-bicyclo [2,2,1] hept-2Z-ene (30 g) is dissolved in 200 ml of ethanol and 0.3 g of 10% palladium on charcoal is added. The mixture is vigorously stirred in 1 atmosphere of hydrogen gas at room temperature. 1 molar equivalent of hydrogen gas is absorbed and the product is then isolated by removal of the catalyst by filtration through a Celite (trade name) pad, followed by evaporation of the filtrate to give a quantitative yield of the title compound as an oil, b.p. 105—110°C/1.5 mm.

4. *5-endo-Hydroxymethyl-6-exo-diethoxymethyl-bicyclo [2,2,1] heptane*

The ester (3A) (27 g) is added in ether to a 10% excess of lithium aluminium hydride (2.1 g) in ether with stirring at reflux temperature. The mixture is boiled for 1 hour after the addition and is then quenched by the addition of wet ether followed by 5% aqueous sodium hydroxide to precipitate aluminum salts. The colourless organic phase is dried over magnesium sulphate, filtered and evaporated to give the title compound as an oil (20 g, 91%).

5. *5-endo-Cyanomethyl-6-exo-diethoxymethyl-bicyclo [2,2,1] heptane*

The alcohol (4) (20 g) in a minimum volume of dry pyridine is added slowly to 20 g of p-toluensulphonyl chloride in 130 ml dry pyridine with stirring at 0°C. The mixture is kept at 5°C overnight and then poured into a water/ice mixture. The resulting precipitate is filtered off and dried to give the tosylate ester of the alcohol in 85% yield as an off-white solid, m.p. 84—86°C (dec).

The tosylate (14 g) in 15 ml dimethyl sulphoxide is added to 5 g of dry potassium cyanide in 20 ml dimethyl sulphoxide. The mixture is stirred under nitrogen and the temperature slowly raised over 1 hour to 110°C. After 5 hours the reaction mixture is cooled and poured into water. The product is isolated by ether extraction, and purified by distillation to give the title compound (7.8 g, 90%), b.p. 115—126°C/1.5 mm.

6. *6-exo-Diethoxymethyl-5-endo-formylmethyl-bicyclo [2,2,1] heptane*

The cyano compound (5) (20 g) is stirred at −15°C in 200 ml dry toluene under nitrogen. Di-isobutylaluminium hydride (113 ml of a 1M solution in hexane) is added to the substrate over 25 minutes and the mixture allowed to reach room temperature. After 1 hour, methanol (30 ml) is cautiously added, followed by 400 ml of saturated aqueous sodium hydrogen tartrate. The mixture is stirred and heated at 40°C for 2 hours. The upper organic layer is separated and the aqueous phase

further extracted with ethyl acetate. The combined organic solutions are dried ($MgSO_4$) and the solvent removed to give a yellow oil. This is chromatographed on Florisil (trade name) in benzene to give the pure title compound as a colourless oil (17.2 g, 85%), $\nu_{max}$ (film): 1725 $cm^{-1}$.

7. *5-endo-(6'-Carboxyhex-2'Z-enyl)-6-exo-diethoxymethyl-bicyclo [2,2,1] heptane*

(4-Carboxy-n-butyl)-triphenylphosphonium bromide (23.3 g) is dried at 75°C under vacuum for 2.5 hours. The resulting white solid is then cooled, the vacuum released to dry nitrogen, and 30 ml of dimethyl sulphoxide is added. A 2M solution of dimesyl sodium in dimethyl sulphoxide (50 ml) is added slowly while the mixture is maintained at 25°C with a water bath. After 15 minutes the aldehyde (6) (5.0 g) is added to the deep red yield thus produced. The mixture is stirred overnight and then the solvent is removed at 55—60°C under vacuum. The residue is dissolved in water, and the aqueous phase is extracted with ether and is then carefully acidified to pH 4 with 2N HCl. The precipitate is extracted into ether and the ethereal solution is dried and concentrated to give the title compound as an oil (3.7 g, 55%).

8. *5-endo-(6'-Carboxyhex-2'Z-enyl)-6-exo-formyl-bicyclo [2,2,2] heptane*

The acid acetal (7) (1.8 g) is dissolved in 200 ml chloroform and 50 ml of concentrated hydrochloric acid is added to form a two phase system. This mixture is vigorously stirred for 90 minutes and then extracted with ether and the ethereal solution dried and concentrated. The residual oil is purified by silicic acid chromatography, the oil being applied to the column (prepared by slurrying 10 g of Unisil silicic acid — trade name, Clarkson Chemical Co. USA — in hexane and pouring into a glass chromatography column) in hexane and elution being carried out with increasing proportions of diethyl ether in hexane up to pure diethyl ether. The chromatography gives the title compound as a colourless oil (1.4 g, 83%), $\nu_{max}$(film): 795,1715 (broad), 2700 $cm^{-1}$; $\delta$ ($CDCl_3$): 1.2 to 2.6 (18H,m), 5.4 (2H,m), 9.6 (1H,d). *Note:* Care should be taken to avoid contact of this compound with methanol since it very readily forms the dimethyl acetal.

(B) Preparation of 5-end-(6′-Carboxyhex-2′Z-enyl)-6-exo-formylbicyclo [2,2,1] hept-2Z-ene

1,2,3 *5-endo-Ethoxycarbonyl-6-exo-diethoxymethyl-bicyclo [2,2,1] hept-2Z-ene*

Maleinaldehydic acid pseudo-ethyl ester is prepared as described in section (1) of (A) above and reacted with cyclopentadiene in a Diels-Alder reaction as described in section (2). The Diels-Alder adduct is then treated with ethanol under acidic conditions as described in section (3) to give 5-endo-ethoxycarbonyl-6-exo-diethoxymethyl-bicyclo 2,2,1 hept-2Z-ene.

4. *5-endo-Hydroxymethyl-6-exo-diethoxymethyl-bicyclo [2,2,1] hept-2Z-ene*

The ester/acetal (3) is added in ether to lithium aluminium hydride (10% excess) in ether with stirring at reflux temperature. After the addition, the mixture is boiled for a further 1 hour. The reaction is quenched with wet ether and then 5% aqueous sodium hydroxide to precipitate aluminium. The colourless organic phase is filtered, dried over anhydrous potassium carbonate, and the resulting alcohol (85—90% yield) used directly in the next stage.

5. *5-endo-Cyanomethyl-6-exo-diethoxymethyl-bicyclo [2,2,1] hept-2Z-ene*

The alcohol (4) (7 g) in 15 ml dry pyridine is added slowly at 0°C to p-toluenesulphonyl chloride (7.5 g) in pyridine (45 ml). The mixture is kept overnight at 10°C and then quenched by pouring over ice with vigorous shaking. The product is extracted with ether, washed consecutively with water, 0.1 M sodium carbonate and brine, and then dried ($K_2CO_3$) and the solvent removed to give the tosylate ester of the alcohol as a colourless oil in high yield.

The tosylate ester (12 g) in dimethyl sulphoxide (15 ml) is added with stirring to potassium cyanide (3 g) in dimethyl sulphoxide (20 ml). The mixture is heated to 100°C under nitrogen for 6 hours and is then cooled, poured into water and the product taken into ether. The solvent is removed and the residue distilled to give title compound as an oil (6.6 g, 88%), b.p. 112—124°C/1.8 mm.

6. *6-exo-Diethoxymethyl-5-formylmethyl-bicyclo [2,2,1] hept-2Z-ene*

Di-isobutylaluminium hydride (25 ml of 1M solution in hexane) is added with stirring over a 10 minute period to the cyano compound (5) (5.0 g) in dry toluene (70 ml) at —15°C under nitrogen. After a further 1 hour at room temperature the reaction is terminated by the addition with caution of methanol (6 ml), followed by saturated aqueous sodium hydrogen tartrate (95 ml). The mixture is then stirred and heated at 40°C for two hours. The organic phase is separated and the aqueous layer is further extracted with ethyl acetate, the combined organic solutions being dried and stripped of solvent to give the product as a yellow oil. Chromatography on Florisil in benzene gives the pure title compound as a colourless oil (3.2 g, 63%), $\nu_{max}$ (film): 1725 $cm^{-1}$.

7. *5-endo-(6'-Carboxyhex-2'Z-enyl)-6-exo-diethoxymethyl-bicyclo [2,2,1] hept-2Z-ene*

(4-Carboxy-n-butyl)-triphenylphosphonium bromide (7.0 g) is dried at 75°C under vacuum for 90

minutes. The white solid is cooled, the vacuum is released to dry nitrogen and 10 ml of dimethyl sulphoxide (10 ml) is added followed by 15 ml of a 2M solution of dimesyl sodium in dimethyl sulphoxide. The temperature is maintained at 25°C and the aldehyde (6) (1.5 g) is added to the deep red yield solution. After stirring overnight the solvent is removed at 55—60°C under vacuum. The residue is dissolved in water, extracted with ether, and the aqueous phase carefully acidified to pH 4 with 2N HCl. The mixture is extracted with ether and the ethereal solution dried and concentrated to give the title compound as an oil (1.34 g, 66%).

8. *5-endo-(6'-Methoxycarbonylhex-2'Z-enyl)-6-exo-formyl-bicyclo [2,2,1] hept-2Z-ene*

The acid/acetal (7) (5 g) in ether is treated with excess ethereal diazomethane to form the methyl ester and then the ketal protecting group is removed by dissolving the compound in 215 ml chloroform and adding concentrated hydrochloric acid (55 ml) to form a two-phase system. The mixture is extracted with ether and the ethereal solution dried and concentrated to give the title compound as an oil (3.38 g, 90%).

*Note:* Care should be taken to avoid contact of this compound with methanol since it very readily forms the dimethyl acetal.

(C) Preparation of 5-endo-(6'-Carboxyhexyl)-6-exo-formyl-bicyclo [2,2,1] heptane

5-endo-(6'-Carboxyhex-2'Z-enyl)-6-exo-diethoxymethyl-bicyclo [2,2,1] heptane is prepared as described in section (7) of (A) above. This acid/acetal (300 mg) is stirred with 10% palladium charcoal (50 mg) in absolute ethanol (10 ml) for 30 minutes whilst continuously passing hydrogen gas through the suspension. The catalyst is removed by filtration through a Whatman No. 550 filter disc and the ethanol is then removed in vacuo. The oily residue of 5-endo-(6'-carboxyhexyl)-6-exo-diethoxymethyl-bicyclo [2,2,1] heptane is dissolved in $CHCl_3$ (50 ml), 2N aqueous hydrochloric acid (50 ml) is added, and the two phase system is stirred for 6 hours at room temperature. Water (100 ml) is then added, followed by diethyl ether (150 ml) and after vigorous shaking the organic phase is separated. The aqueous phase is extracted with a further 150 ml of diethyl ether and the two ether extracts are combined. Evaporation of the diethyl ether from the dried solution gives 5-endo-(6'-carboxyhexyl)-6-exo-formyl-bicyclo [2,2,1] heptane as an oil (152 mg), $\nu_{max}$(film): 1715 $cm^{-1}$ (broad); $\delta$ ($CDCl_3$): 1.1—2.6 (22H, aliphatic H), 9.6 (d, 1H, C*H*O), 10.0 (broad, COO*H*); M.S. (methyl ester): $M^+/M^+ + 1$ 266/267 — single peak.

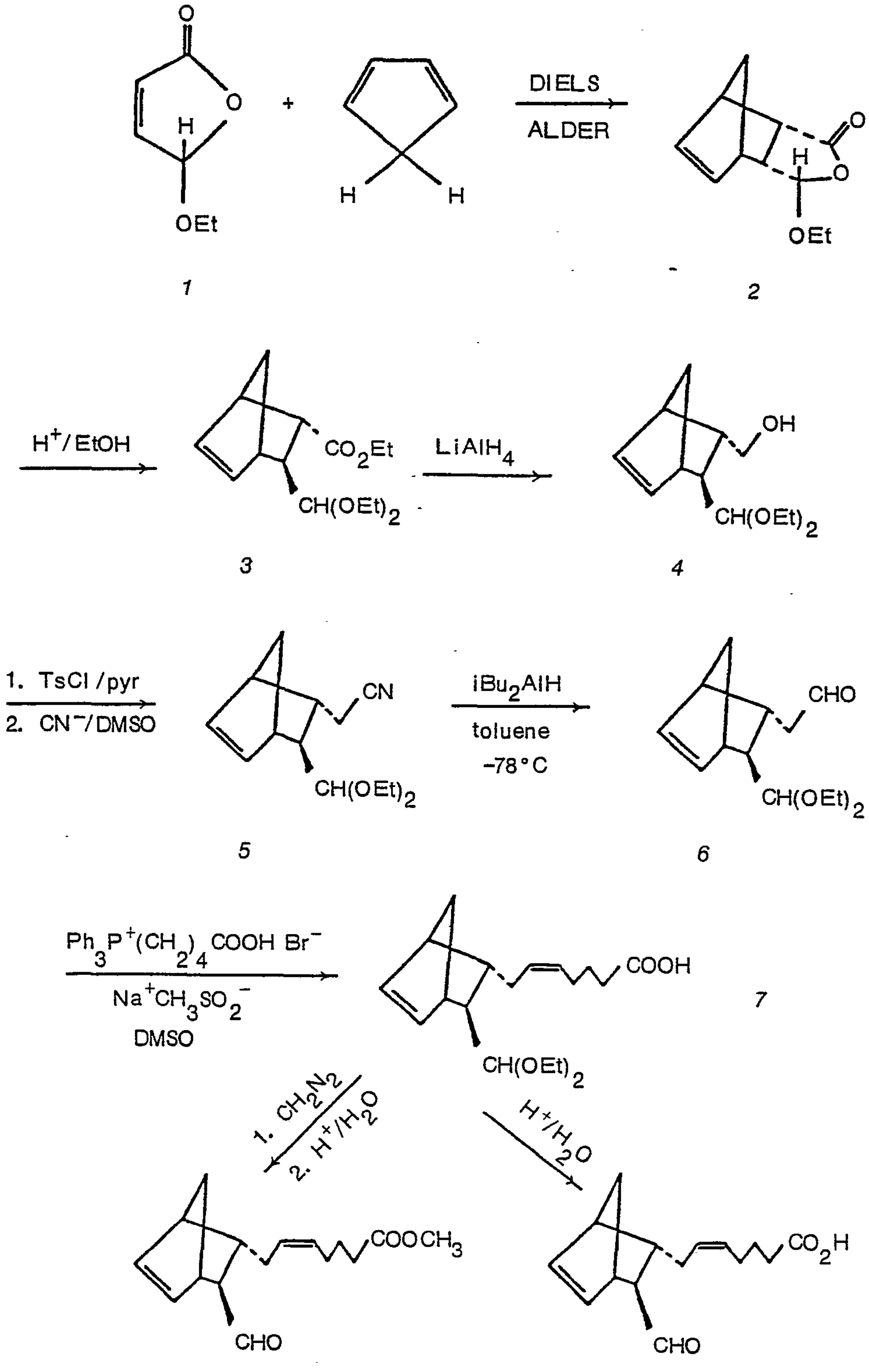


1 2 3 4 5 6 7 8 8'

## Claims

1. A compound being a bicyclo [2,2,1] heptane or bicyclo [2,2,1] hept-2Z-ene which is substituted at the 5-position by a 6-carboxyhex-2-enyl group or a modification thereof in which the group is altered by one, or an appropriate combination of two or more, of the following:
(a) a reduction of the double bond optionally accompanied by replacement of a carbon atom at the 1, 2 or 3 position by a sulphur or oxygen atom; (b) alteration of the position of the double bond;
(c) shortening or lengthening of the carbon chain by one or two methylene groups; and (d) formation of an amide, ester or salt derivative of the carboxy group; and at the 6-position by a group

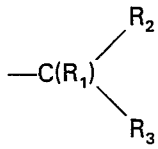

in which $R_1$ represents an aliphatic hydrocarbon residue or an aliphatic hydrocarbon residue substituted directly or through an oxygen or sulphur atom by an aromatic residue, $R_2$ represents hydrogen and $R_3$ represents a hydroxy group, or $R_2$ and $R_3$ together represent the oxygen atom of a carbonyl group.

2. A compound according to Claim 1, in which any modification of the 6-carboxyhex-2-enyl group of type (c) is a shortening or lengthening of the carbon chain by one methylene group.

3. A compound according to Claim 1, in which the substituent at the 5-position is a 6-carboxyhex-2-enyl group or a derivative thereof formed at the carboxy group.

4. A compound according to Claim 1, in which the substituent at the 5-position is a 6-carboxyhexyl group or a derivative thereof formed at the carboxy group.

5. A compound according to any of Claims 1 to 4, in which the 5-substituent terminates in a free carboxy group.

6. A compound according to any of Claims 1 to 4, in which the 5-substituent terminates in an ester derivative of the carboxy group.

7. A compound according to any of the preceding claims, in which the substituent at the 6-position is a group —$C(R_1)=O$ in which $R_1$ represents a residue as defined in Claim 1.

8. A compound according to any of the preceding claims, in which $R_1$ represents an aliphatic hydrocarbon residue.

9. A compound according to Claim 8, in which $R_1$ represents an aliphatic hydrocarbon residue of 1 to 5 carbon atoms.

10. A compound according to any of Claims 1 to 6, in which the substituent at the 6-position is a group

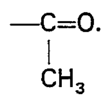

11. A compound according to any of the preceding claims being a bicyclo [2,2,1] hept-2Z-ene.

12. A compound according to any of claims 1—10 being a bicyclo [2,2,1] heptane.

13. A compound according to any of the preceding claims, in which the configuration about any double bond in the 5-substituent is *cis*.

14. A compound according to any of the preceding claims, in which the 5- and 6-substituents are in *trans* relationship.

15. A compound according to any of the preceding claims, in which the 5-substituent is oppositely disposed to the bridging methylene group.

16. 5-endo-(6′-Carboxyhex-2′Z-enyl)-6-exo-(1′-hydroxyethyl)bicyclo [2,2,1] heptane.

17. 5-endo-(6′-Carboxyhex-2′Z-enyl)-6-exo-acetyl-bicyclo [2,2,1] heptane.

18. 5-endo-(6′-Carboxyhexyl)-6-exo-acetyl-bicyclo [2,2,1] heptane.

19. 5-endo-(6′-Carboxyhex-2′Z-enyl)-6-exo-acetyl-bicyclo [2,2,1] hept-2Z-ene.

20. 5-endo-(6′-Carboxyhexyl)-6-exo-acetyl-bicyclo [2,2,1] hept-2Z-ene.

**Revendications**

1. Composé qui est un bicyclo [2,2,1] heptane ou un bicyclo [2,2,1] hept-2Z-ène et qui est substitué en position 5 par un groupe 6-carboxyhexe-2-ényle ou une varianete ou modification de ce groupe dans laquelle le groupe est modifié par une, ou par une combinaison appropriée de deux ou plusieurs, des opérations suivantes:

(a) une réduction de la double liaison, éventuellement accompagnée d'un remplacement d'un atome de carbone en position 1, 2, 3 par un atome de soufre ou d'oxygène; (b) modification de la position de la double liaison; (c) raccourcissement ou allongement, d'un ou deux groupes méthylène, de la chaîne carbonée; et (d) formation d'un dérivé de type amide, ester ou sel du groupe carboxyle; et qui est substitué en position 6 par un groupe

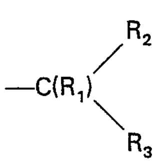

dans lequel $R_1$ représente un radical d'hydrocarbure aliphatique ou un radical d'hydrocarbure

aliphatique substitué, directement ou par l'intermédiaire d'un atome d'oxygène ou de soufre, par un radical aromatique, $R_2$ représente de l'hydrogène et $R_3$ représente un groupe hydroxyle, ou bien $R_2$ et $R_3$ pris ensemble représentent l'atome d'oxygène d'un groupe carbonyle.

2. Composé selon la revendication 1, dans lequel toute modification éventuelle du groupe 6-carboxyhex-2-ényle de type (c) est un raccourcissement ou un allongement, d'un groupe méthylène, de la chaîne carbonée.

3. Composé selon la revendication 1, dans lequel le substituant en position 5 est un groupe 6-carboxyhex-2-ényle ou un dérivé de ce groupe formé sur le groupe carboxyle.

4. Composé selon la revendication 1, dans lequel le substituant en position 5 est un groupe 6-carboxyhexyle ou un dérivé de ce groupe formé sur le groupe carboxyle.

5. Composé selon l'une quelconque des revendications 1 à 4, dans lequel le substituant fixé en position 5 se termine par un groupe carboxyle libre.

6. Composé selon l'une quelconque des revendications 1 à 4, dans lequel le substituant fixé en position 5 se termine par un ester dérivant du groupe carboxyle.

7. Composé selon l'une quelconque des revendications précédentes, dans lequel le substituant en position 6 est un groupe —$C(R_1)$=O, dans lequel $R_1$ représente un radical tel que défini à la revendication 1.

8. Composé selon l'une quelconque des revendications précédentes, dans lequel $R_1$ représente un radical d'hydrocarbure aliphatique.

9. Composé selon la revendication 8, dans lequel $R_1$ représente un radical d'hydrocarbure aliphatique ayant 1 à 5 atomes de carbone.

10. Composé selon l'une quelconque des revendications 1 à 6, dans lequel le substituant en position 6 est un groupe

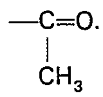

11. Composé selon l'une quelconque des revendications précedentes, qui est un bicyclo [2,2,1] hept-2Z-ène.

12. Composé selon l'une quelconque des revendications 1 à 10, qui est un bicyclo [2,2,1] heptane.

13. Composé selon l'une quelconque des revendications précédentes, dans lequel la configuration autour de n'importe quelle double liaison présente dans le substituant fixé en position 5 est en cis.

14. Composé selon l'une quelconque des revendications précédentes, dans lequel les substituants fixés en position 5 et 6 sont en relation trans.

15. Composé selon l'une quelconque des revendications précédentes, dans lequel le substituant fixé en position 5 est disposé à l'opposé du groupe méthylène de pontage.

16. Le 5-endo-(6'-carboxyhex-2'Z-ényl)-6-exo-(1'-hydroxyéthyl)-bicyclo [2,2,1] heptane.

17. Le 5-endo-(6'-carboxyhex-2'Z-ényl)-6-exo-acétyl-bicyclo [2,2,1] heptane.

18. Le 5-endo-(6'-carboxyhexyl)-6-exo-acétyl-bicyclo [2,2,1] heptane.

19. Le 5-endo-6'-carboxyhex-2'Z-ényl)-6-exo-acétyl-bicyclo [2,2,1] hept-2Z-ène.

20. 5-endo-(6'-Carboxyhexyl)-6-exo-acétyl-bicyclo [2,2,1] hept-2Z-ène.

## Patentansprüche

1. Bicyclo [2,2,1] heptan oder Bicyclo [2,2,1] hept-2Z-en, das in der 5-Stellung durch eine 6-Carboxyhex-2-enyl-gruppe oder eine derartige modifizierte Gruppe substituiert ist, wobei die Gruppe durch eine oder eine geeignete Kombination von zwei oder mehreren der folgenden Modifizierungen

a) Reduktion der Doppelbindung, ggf. Ersatz eines Kohlenstoffatoms in der 1, 2 oder 3-Stellung durch ein Schwefel- oder Sauerstoffatom;

b) Änderung der Stellung der Doppelbindung;

c) Verkürzung oder Verlängerung der Kohlenstoffkette durch ein oder zwei Methylengruppen und

d) Bildung eines Amid-, Ester- oder Salzderivats der Carboxygruppe modifiziert ist und welches in der 6-Stellung durch eine Gruppe

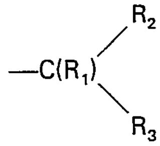

substituiert ist, worin $R_1$ einen aliphatischen Kohlenwasserstoffrest oder einen aliphatischen Kohlenwasserstoffrest, der direkt oder über ein Sauerstoff- oder Schwefelatom durch einen aromatischen Ring substituiert ist, $R_2$ Wasserstoff, $R_3$ eine Hydroxygruppe bedeuten oder worin $R_2$ und

$R_3$ zusammen ein Sauerstoffatom einer Carbonylgruppe bedeuten.

2. Verbindung nach Anspruch 1, dadurch gekennzeichnet, daß die Modifizierung der 6-Carboxyhex-2-enyl-gruppe des Typs (c) eine Verkürzung oder eine Verlängerung der Kohlenstoffgruppe durch eine Methylengruppe ist.

3. Verbindung nach Anspruch 1, dadurch gekennzeichnet, daß der Substituent in der 5-Stellung eine 6-Carboxyhex-2-enyl-gruppe oder ein Derivat davon, welche an die Carboxygruppe gebildet wurde, ist.

4. Verbindung nach Anspruch 1, dadurch gekennzeichnet, daß der Substituent in der 5-Stellung eine 6-Carboxyhexylgruppe oder ein Derivat davon, das mit der Carboxygruppe gebildet wurde, ist.

5. Verbindung nach einem Der Ansprüche 1 bis 4, dadurch gekennzeichnet, daß der 5-Substituent mit einer freien Carboxygruppen beendigt wird.

6. Verbindung nach einem der Ansprüche 1 bis 4, dadurch gekennzeichnet, daß der 5-Substituent mit einem Esterderivat der Carboxygruppe beendigt wird.

7. Verbindung nach einem der vorhergehenden Ansprüche, dadurch gekennzeichnet, daß der Substituent in der 6-Stellung eine Gruppe $—C(R_1)=O$ ist, worin $R_1$ einen Rest bedeutet, wie er in Anspruch 1 definiert wurde.

8. Verbindung nach einem der vorhergehenden Ansprüche, dadurch gekennzeichnet, daß $R_1$ eine aliphatischen Kohlenwasserstoffrest bedeutet.

9. Verbindung nach Anspruch 8, dadurch gekennzeichnet, daß $R_1$ einen aliphatischen Kohlenwasserstoffrest mit 1 bis 5 Kohlenstoffatomen bedeutet.

10. Verbindung nach einem der Ansprüche 1 bis 6, dadurch gekennzeichnet, daß der Substituent in der 6-Stellung einer Gruppe

$$\begin{array}{l}—C=O\\ \quad |\\ \ \ CH_3\end{array}$$

bedeutet.

11. Verbindung nach einem der vorhergehenden Ansprüche, dadurch gekennzeichnet, daß sie ein Bicyclo [2,2,1]hept-2Z-en ist.

12. Verbindung nach einem der Ansprüche 1 bis 10, dadurch gekennzeichnet, daß sie ein Bicyclo [2,2,1] heptan ist.

13. Verbindung nach einem der vorgehenden Ansprüche, dadurch gekennzeichnet, daß die Konfiguration an der Doppelbindung in dem 5-Substituenten cis ist.

14. Verbindung nach einem der vorhergehenden Ansprüche, dadurch gekennzeichnet, daß die 5- und 6-Substituenten in Transbeziehung zueinander stehen.

15. Verbindung nach einem der vorhergehenden Ansprüche, dadurch gekennzeichnet, daß der 5-Substituent entgegengesetzt zu der Brückenmethylengruppe vorhanden ist.

16. 5-Endo-(6′-Carboxyhex-2′Z-enyl)-6-exo-(1′-hydroxyethyl)-bicyclo [2,2,1] heptan.

17. 5-Endo-(6′-Carboxy-2′Z-enyl)-6-exo-acetyl-bicyclo [2,2,1] heptan.

18. 5-Endo-(6′-Carboxyhexyl)-6-exo-acetyl-bicyclo [2,2,1] heptan.

19. 5-Endo-(6′-Carboxyhex-2′Z-enyl)-6-exo-acetyl-bicyclo [2,2,1] hept-2Z-en.

20. 5-Endo-(6′-Carboxyhexyl)-6-exo-acetyl-bicyclo [2,2,1] hept-2Z-en.